# EUROPEAN PATENT SPECIFICATION

(11) **EP 1 406 550 B1**
(45) Date of publication and mention of the grant of the patent: **22.09.2010**
(21) Application number: 02733763.3
(22) Date of filing: 26.06.2002
(51) Int. Cl.: A61C 8/00, A61L 27/32, A61L 27/56

(54) **IMPLANT**
IMPLANTAT
IMPLANT

(30) Priority: 04.07.2001 SE 0102388
(43) Date of publication of application: 14.04.2004
(73) Proprietor: Nobel Biocare AB (publ), 402 26 Göteborg (SE)
(72) Inventor: HALL, Jan, S-416 80 Göteborg (SE)
(74) Representative: Byström, Kurt Linus
(86) International application number: PCT/SE2002/001254
(87) International publication number: WO 2003/003936

(56) References cited:
- WO-A1-00/72776
- WO-A1-98/48862
- WO-A1-02/078759

## Description

The present invention relates to an implant comprising or consisting of a tissue-compatible material and having one or more surfaces which can be applied in or on a tissue area and/or bone growth area or bone growth areas, for example the jaw bone. One or more surfaces of said surfaces are provided with a pore arrangement in material oxide layers of substantial thickness, by which is meant thicknesses, for example, within the range of 1 - 20 µm. The tissue-compatible material is preferably titanium, but can also be a titanium alloy, tantalum, zirconium, etc.

It is already known to produce implants with oxide layers with substantial thicknesses and porosities. Reference may be made here to the Swedish patent application 9901974-7 filed by the same Applicant and to areas of the prior art mentioned therein. It is thus known to form the porous and thick layers by using various methods, for example the Ti plasma method, shot-peening, etching, anodic oxidation, etc.

In connection with implants, it is also known to use bone-growth-stimulating agents or coatings of CaP (calcium phosphate compounds), for example in the form of HA (hydroxyapatite). The application can be carried out, for example, by a sputtering method in the form of so-called RF sputtering with subsequent heat treatment. Reference is made, inter alia, to PCT specification WO 98/48862 and to pertinent parts of the prior art mentioned therein.

In connection with implants in dental contexts, for example in the jaw bone, there is a need to achieve a high degree of individuality of treatment on account of the presence of widely varying jaw bone structures, physical responses, degrees of acidity, etc. There is therefore a need to be able to obtain the desired implant properties in different individuals who may react very differently to bone-growth-stimulating materials or substances. By choosing the porosity and thickness of the oxide layer, the implant material can itself stimulate the process of incorporation, and the use of bone-growth-stimulating agents can be combined with the choice of porosity and thickness of the oxide layer in order to afford further stimulation. The invention aims to solve this problem, inter alia, and proposes implant structures which are adapted to different treatment situations.

WO98/48862 discloses a calcium phosphate coated implant element. The coating is applied on a well-documented surface in order to combine properties for rapid bone growth during the healing phase and properties for a guaranteed long-term stability during clinical loading conditions.

WO00/72776 discloses a layer arranged on an implant for bone or tissue structure. The layer is designed with a channel network which gives the layer a substantial porosity.

WO02/078759 discloses a bioactive surface layer. A variable portion of the porous surface layer is comprised of calcium phosphate phases.

It is expedient if means and methods which are known per se can be used so that the desired results are unambiguous. The invention also aims to solve this problem.

According to one embodiment of the invention, it is expedient to be able to determine the relation between the structures of the oxide layer and the choice of the CaP layer. The invention solves this problem too and is primarily intended for persons who are to receive implants with very porous and thick oxide layers, while at the same time the use of bone-growth-stimulating material is intended to be minimal.

That which can mainly be regarded as characterizing an implant according to the invention is that the pore arrangement in question is coated with one or more thin layers made up of calcium phosphate compound(s), for example one or more layers with a total thickness of a few Angstroms and ca. 5 µm.

The implant 18 has one or more surfaces 18a, 18b, 18c which can be applied in or on tissue and/or bone growth areas 11.

The thickness or thicknesses or quantity or quantities of the layer or layers can be varied as a function of the depth of the porous oxide layer. Thus, the CaP layer in the outer parts of the oxide layer can have a greater thickness compared to the case of the inner parts of the oxide layer. The pore arrangement can be arranged such that, in the particular application method, it receives CaP with thin coating layers in its inner parts and greater or thicker coating layers in its outer parts. In another embodiment, the layers can be varied by means of the method of application.

In said embodiments, the degree of crystallization of each layer will also be chosen so that CaP can be released to the surrounding bone substance according to the desired pattern as a function of the chosen time aspect. The oxide layer will also have a surface roughness, degree of porosity and pore sizes with values known per se.

By means of what has been indicated above, an implant structure is obtained which covers an allocated niche within the overall treatment area. Proven and practicable techniques with known or expected results can be used and obtained, and the implant in question can form part or parts of the total range offered by the supplier.

A presently proposed embodiment of an implant according to the invention will be described below with reference to the attached drawings, in which:
- Figure 1: shows diagrammatically in vertical section, and on a greatly enlarged scale, an oxide layer structure on an implant, where CaP coatings have thickness which vary as a function of their positions in the pores of the oxide layer,
- Figure 2: shows, in an outline diagram, the implant in an apparatus which creates oxide layers of great porosity and great thickness on the implant,
- Figure 3: shows, in a side view and in outline form,
- Figure 4: complementary or alternative embodiments for creating the porous and thick oxide layers, shows, in a vertical view, application of CaP layers, for example HA layers, in an apparatus,
- Figure 5: shows, in an outline diagram, the heat treatment equipment for the implant which in accordance with Figure 4 has been coated with CaP layers or HA layers, and
- Figure 6: shows in outline form an implant with a thick and porous layer coated with CaP coating.

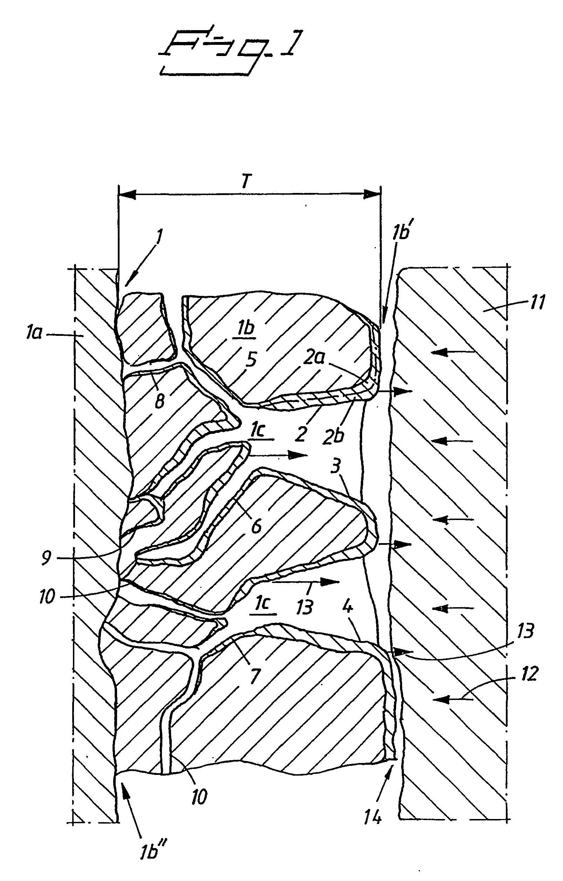

In Figure 1, in an outline diagram, parts of an implant are indicated by reference number 1. The structure of the implant can be of a type known per se and will therefore not be described in detail here. In accordance with the invention, the implant body 1a comprises an oxide layer 1b of substantial thickness T. The layer is additionally highly porous and the pore arrangement is indicated by 1c. Thin layers of CaP, which in the illustrative embodiment can principally consist of HA layers, are applied in the pores. According to a preferred embodiment of the invention, the last-mentioned layers vary in thickness as a function of their positions in the oxide layer 1b. In a preferred embodiment, the CaP layers can in principle have greater thicknesses on the outer surface 1b' of the layer 1b compared to the inner parts of the layer which have been indicated by 1b". Said variation can decrease stepwise or continuously. In Figure 1, outer CaP layers have been indicated by 2, 3, 4, and layers which are located in the central parts of the oxide layer 1b have been indicated by 5, 6 and 7. Inner thinner CaP layers have been indicated by 8, 9 and 10. Each layer can consist of a homogeneous layer or can comprise or be composed of two or more layers 2a, 2b.

The CaP layers can have the same or different degrees of crystallization. However, it is important here that the respective degree of crystallization is chosen as a function of the optimum implant situation or patient situation. Thus, the degree of crystallization can be chosen to be slightly greater than 0% and the upper values of the degrees of crystallization are preferably chosen to be about 50%. It may be possible, completely or in part, to use degrees of crystallization of up to 75 - 100%. Thus, the lowermost layer can be given a degree of crystallization within the range of 75 - 100%. The degrees of crystallization are calculated on the basis of the degree of crystallization 0% which relates to the radiologically amorphous surface where the particle size is at most ca. 50 nm.

In accordance with Figure 1, the implant 1 is anchored in a bone, preferably a jaw bone 11. Body substances 12 begin to act on or are released onto the implant after the implant has been implanted in the jaw bone for a certain time. This means that the CaP layers are released, see the arrows 13, and the body substances and the released CaP substance are mixed via the transition area 14 between jaw bone and implant. The exchange of the substances 12 and 13 stimulates the bone growth in the jaw bone 11 and at the same time the incorporation in the porous layer 1b is made more effective. From the start, therefore, the mechanical and porous layer structure is used to stimulate bone growth and incorporation. By means of the described arrangement, the surrounding bone can be fed with CaP or equivalent during a time period which is determined by layer thickness, porosity and CaP structure.

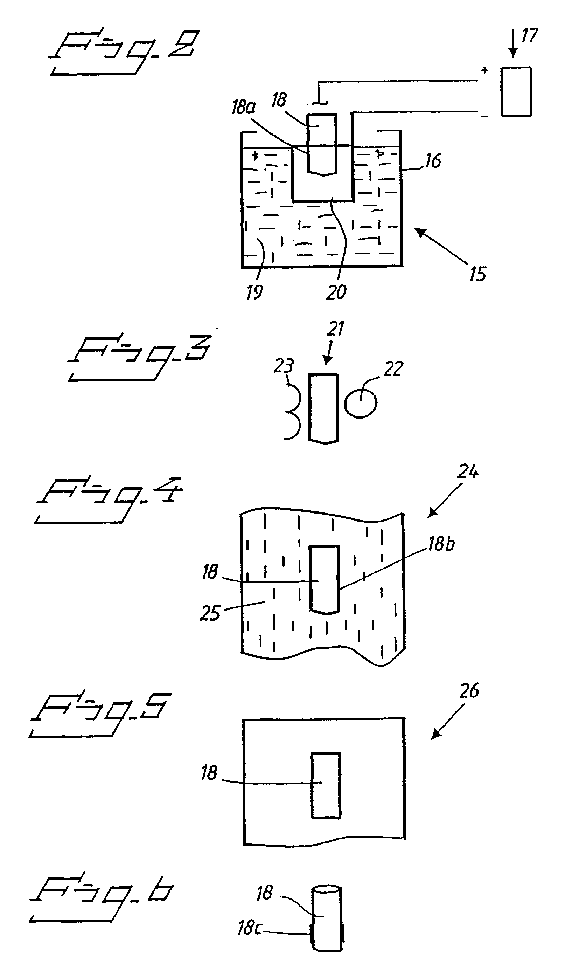

In accordance with Figure 2, the thick and porous oxide layers of the implant can be effected by so-called anodic oxidation (electrochemical method). For this purpose, a vessel 16 is used and an electrical arrangement 17 via which the electrochemical method is carried out. Since the method is already known per se, it will not be described in detail here and is only explained in principle. An implant 18 is lowered into an electrolyte 19 in said vessel and the plus and minus poles are connected to the implant and a unit 20 via which the implant is immersed in the electrolyte 19. The unit 20 can be a cathode. The implant can be provided with the porous and thick oxide layer in whole or in part. The implant which normally has an outer thread 18a or threads can be provided with said layers on these threads. Those parts which are not to be provided with an oxidized layer are masked in a manner known per se. The filling of the oxide layer, for example the titanium oxide layer, and its final properties are affected by a number of parameters in the process, among which there may be mentioned the composition of the electrolyte and its temperature, the voltage applied and the current, the electrode geometries and treatment times. In one embodiment, the oxide layer can have a surface roughness within a range of 0.4 - 5 µm. The high degree of porosity means a number of pores/cm² of 1 x 10⁷ - 1 x 10¹⁰. The pore sizes are also of importance and pores with diameter sizes within the range of 0.1 - 10 µm are chosen. As regards the total pore volume, this can be chosen within the range of 5 x 10⁻² and 10⁻⁵ cm³.

In accordance with Figure 3, complementary or alternative methods for layer production can be chosen, examples of such methods which may be mentioned being shot-peening which has been symbolized by 22, and etching which has been symbolized by 23. As a complement or alternative, plasma spraying can be used.

After the oxide layer formation on the implant in question, CaP layers will be applied in accordance with the invention. In the present case, one or more CaP layers are to be applied to the implant. In accordance with Figure 4, the implant is placed in a chamber 24 after any demasking has been carried out.. The surface parts 18b of the implant must be completely or partially provided with a film-like or layer-like coating or coatings. The coating application is carried out in the chamber 24 by so-called RF sputtering or magnetron sputtering in a manner known per se, see for example cited WO 98/48862. One or more calcium phosphate compounds 25 are applied to the surface or surface parts of the implant in the chamber 24.

In accordance with Figure 5, the implant or its relevant surface parts are heat-treated in an oven 26. Temperatures of 600°C, for example, and treatment times in saturated water vapour can be chosen in accordance with known techniques. The aim of the heat treatment is to completely or partially crystallize the applied coating in accordance with the above.

Figure 6 is intended to show the finished implant, where the implant 18 has one or more coatings 18c in accordance with that described in relation to Figure 1. Said coating of CaP has a thickness within the range of a few Angstroms and up to ca. 5 µm.

The implant 18 has one or more surfaces 18a, 18b, 18c which can be applied in or on tissue and/or bone growth areas 11.

The invention is not limited to the embodiment shown above by way of example, and instead it can be modifies within the scope of the attached patent claims and the inventive concept.

## Claims

1. An implant (8) comprising tissue-compatible material, preferably titanium, and having one or more surfaces (18a, 18b, 18c), one or more surfaces of said surfaces being provided with an oxide layer (1b) of substantial thickness, for example 2-20 µm, having a pore arrangement (1c), **characterized in that** the oxide layer of surface parts of the implant is completely or partially coated with at least one calcium phosphate (CaP) layer, for example one or more layers with a total thickness within the range of a few Angstroms and 5 µm, applied as a film-like or layer-like coating.

2. The implant as claimed in patent claim 1, **characterized in that** the thickness or quantity of the calcium phosphate compound layer varies as a function of the depth or thickness (T) of the oxide layer (1b).

3. The implant as claimed in patent claim 1 or 2, **characterized in that** the CaP layer has a greater thickness in outer parts (1b) of the oxide layer compared to the case of inner parts (1b") of the oxide layer.

4. The implant as claimed in any of the preceding patent claim, **characterized in that** each layer of calcium phosphate compound (s) (CaP) consists of substance sputtered onto the pore system.

5. The implant as claimed in any of the preceding patent claims, **characterized in that** the oxide layer has a surface roughness within a range of 0.4-5 µm.

6. The implant as claimed in any of the preceding patent claims, **characterized in that** the oxide layer is highly or substantially porous with a pore number of 1 x 10⁷ - 1 x 10¹⁰ pores/cm².

7. The implant as claimed in any of the preceding patent claims, **characterized in that** each implant surface mainly has pores with diameter sizes within the range of 0.1-10 µm, and/or **in that** the total pore volume is within a range of 5 x 10⁻² and 10⁻⁵ cm³.

8. The implant as claimed in any of the preceding patent claims, **characterized in that** one or more layers of the calcium phosphate compound (s) (CaP) have a chosen degree of crystallization within the range of slightly larger than 0% up tao 75-100%, preferably slightly larger than 0% up to about 50%.

9. The implant as claimed in any if the preceding patent claims, **characterized in that** the implant consists of tissue-compatible material.

## Patentansprüche

1. Implantat (18), das gewebeverträgliches Material, bevorzugt Titan, umfasst und eine oder mehrere Oberflächen (18a, 18b, 18c) aufweist, wobei eine oder mehrere Oberflächen der Oberflächen mit einer Oxidschicht (1 b) von substantieller Dicke, z.B. 2-20 µm, versehen ist, mit einer Porenanordnung (1c), **dadurch gekennzeichnet, dass** die Oxidschicht von Oberflächenteilen des Implantats vollständig oder teilweise mit mindestens einer Calciumphosphat (CaP) Schicht beschichtet ist, z.B. einer oder mehreren Schichten mit einer Gesamtdicke innerhalb des Bereichs von wenigen Ångström und 5 µm, die als eine filmartige oder schichtartige Beschichtung aufgebracht ist.

2. Implantat gemäß Patentanspruch 1, **dadurch gekennzeichnet, dass** die Dicke oder Quantität der Calciumphosphatverbindungsschicht als eine Funktion der Tiefe oder Dicke (T) der Oxidschicht (1 b) variert.

3. Implantat gemäß Patentanspruch 1 oder 2, **dadurch gekennzeichnet, dass** die CaP-Schicht eine größere Dicke in äußeren Teilen (1 b') der Oxidschicht verglichen mit dem Fall von inneren Teilen (1 b") der Oxidschicht aufweist.

4. Implantat gemäß einem der vorhergehenden Patentansprüche, **dadurch gekennzeichnet, dass** jede Schicht von Calciumphosphatverbindung(en) (CaP) aus einer Substanz besteht, die auf das Porensystem gesputtert ist.

5. Implantat gemäß einem der vorhergehenden Patentansprüche, **dadurch gekennzeichnet, dass** die Oxidschicht eine Oberflächenrauheit innerhalb eines Bereichs von 0,4 bis 5 µm aufweist.

6. Implantat gemäß einem der vorhergehenden Patentansprüche, **dadurch gekennzeichnet, dass** die Oxidschicht hoch- oder substantiell porös mit einer Porenzahl von 1 x 10⁷ bis 1 x 10¹⁰ Poren/cm² ist.

7. Implantat gemäß einem der vorhergehenden Patentansprüche, **dadurch gekennzeichnet, dass** jede Implantatoberfläche hauptsächlich Poren mit Durchmessergrößen innerhalb des Bereichs von 0,1 bis 10 µm aufweist und/oder dass das Gesamtporenvolumen innerhalb eines Bereichs von 5 x 10⁻² und 10⁻⁵ cm³ ist.

8. Implantat gemäß einem der vorhergehenden Patentansprüche, **dadurch gekennzeichnet, dass** die eine oder mehreren Schichten aus der Calciumphosphatverbindung(en) (CaP) einen gewählten Kristalüsationgrad innerhalb des Bereichs von etwas größer als 0 % bis zu 75-100 %, bevorzugt etwas größer als 0 % bis zu ungefähr 50 % aufweist.

9. Implantat gemäß einem der vorhergehenden Patentansprüche, **dadurch gekennzeichnet, dass** das Implantat aus gewebeverträglichem Material besteht.

## Revendications

1. Implant (7.8) comprenant un matériau compatible avec le tissu, de préférence du titane, et rayant une ou plusieurs surfaces (18a, 18b, 18c), une ou plusieurs surfaces desdites surfaces étant prévues avec une couche d'oxyde (16) d'épaisseur sensible, par exemple de l'ordre de 2 - 20 µm, ayant un agencement de pore (1c), **caractérisé en ce que** la couche d'oxyde des parties de surface de l'implant est complètement ou partiellement recouverte avec au moins une couche de phosphate de calcium (CaP), par exemple une ou plusieurs couches avec une épaisseur totale comprise entre quelques Angstroms et 5 µm appliquées sous la forme d'un revêtement de type film ou de type couche.

2. Implant selon la revendication 1, **caractérisé en ce que** l'épaisseur ou la quantité de couche de composé de phosphate de calcium varie en fonction de la profondeur ou de l'épaisseur (T) de la couche d'oxyde (1b).

3. Implant selon la revendication 1 ou 2, **caractérisé en ce que** la couche de CaP a une épaisseur plus importante dans les parties externes (lb') de la couche d'oxyde par rapport au cas des parties internes (1b") de la couche d'oxyde.

4. Implant selon l'une quelconque des revendications précédentes, **caractérisé en ce que** chaque couche du (des) composé(s) de phosphate de calcium (CaP) se compose d'une substance pulvérisée sur le système de pore.

5. Implant selon l'une quelconque des revendications précédentes, **caractérisé en ce que** la couche d'oxyde a une rugosité de surface de l'ordre de 0,4 - 5 µm.

6. Implant selon l'une quelconque des revendications précédentes, **caractérisé en ce que** la couche d'oxyde est très ou sensiblement poreuse avec un nombre de pores de 1 x 10⁷ 1 x 10¹⁰ pores/cm².

7. Implant selon l'une quelconque des revendications précédentes, **caractérisé en ce que** chaque surface d'implant a principalement des pores avec des tailles de diamètre de l'ordre de 0,1 - 10 µm, et/ou **en ce que** le volume de pore total est comprise entre 5 x 10⁻² et 10⁻⁵ cm³.

8. Implant selon l'une quelconque des revendications précédentes, **caractérisé en ce qu'**une ou plusieurs couches du (des) composé(s) de phosphate de calcium (CaP) ont un degré choisi de cristallisation compris dans une plage légèrement supérieure à 0 % jusqu'à 75-100 %, de préférence légèrement supérieure à 0 jusqu'à environ 50 %.

9. Implant selon l'une quelconque des revendications précédentes, **caractérisé en ce que** l'implant se compose d'un matériau compatible avec le tissu.
